# EUROPEAN PATENT APPLICATION

(11) **EP 4 477 313 A1**
(43) Date of publication of application: **18.12.2024**
(21) Application number: 23179434.8
(22) Date of filing: 15.06.2023
(51) Int. Cl.: B01L 3/00, A61B 5/08, B01L 9/00, G01N 33/497

(54) **DEVICES AND METHODS RELATED TO GAS STORAGE**

(71) Applicant: Deep Breath Intelligence AG, 6343 Rotkreuz (CH)
(72) Inventor: KELLER, Beat, 8049 Zürich (CH); ROGLER, Simon Tobias, 8003 Zürich (CH); DINKEL, Lucijana, 8047 Zürich (CH); ZWICKY, Christian, 8044 Gockhausen (CH)
(74) Representative: Rutz, Andrea

(57) **Abstract**

A storage assembly (1) for storing a gas, preferably breath gas, comprises a bag (2) being inflatable and configured to contain the gas, an input piece (3) comprising a valve mechanism, a sealing cap (4) being connectable to the input piece (3), the sealing cap (4) comprising a sealing membrane, and a perforation device (5) being insertable into the input piece (3). The perforation device (5) is configured to perforate the sealing membrane and to cause the valve mechanism to be in the open valve state once the perforation device (5) is inserted in the input piece (3). A gauge device (10) for determining a filling state of the bag (2), a method of delivering a gas sample from the bag (2) to an analyzer assembly (80), and an extraction device (20) for controlled extraction of the gas from the bag (2) are also disclosed.

## Description

### TECHNICAL FIELD

The present invention relates to a storage assembly comprising a bag for storing a gas, a gauge device for determining a filling state of the bag, a method of delivering a gas sample to an analyzer assembly from the bag and an extraction device for controlled extraction of the gas from the bag.

### PRIOR ART

Detecting the presence or measuring the content of an analyte in a gas is desirable for many industrial and medical applications. However, due to their volatile nature, gas samples may be difficult to collect, store and handle. In medical research and diagnostics, the analysis of breath gas samples in particular provides a powerful tool to enable early detection of certain medical conditions in a manner that is non-invasive for the patient. Nevertheless, most advanced analysis systems known in the prior art require the patient to directly exhale into the analysis system and therefore require the patient to travel to a medical facility where such advanced analysis systems are usually installed.

Portable breath bags have thus been developed in order to enable patients to provide a breath gas sample from the comfort of their own home, nursing facility or other convenient location.

US 2014/276100 A1 discloses a system for sensing an analyte in a breath sample, the system including a breath bag and a base. The base further includes a flow handling system and an optical subsystem for sensing a change in an optical characteristic. The breath bag contains a breath sample inlet fitted with a mouthpiece. In one embodiment, the breath sample inlet comprises a one-way valve. The breath sample inlet is designed to mate with a breath bag receiver on the base and the breath bag receiver is equipped with fingers or protrusions designed to open the one-way valve. Attaching the breath bag to the base allows the fingers or protrusions to open the one-way valve so that the contents of the breath bag can be removed by a pump of the base.

No protection of the breath sample inlet is disclosed in US 2014/276100 A1, thus breath gas may inadvertently be lost if the one-way valve is being tampered with after the bag has been filled. Furthermore, the breath sample inlet may easily be contaminated, in particular if the breath bag is stored for longer periods of time, and the contaminations may then directly enter the base, i.e. the analyzer device, and contaminate the latter. In addition, the arrangement disclosed in US 2014/276100 A1 has the disadvantage that the breath bag has to be specifically designed to mate with the breath bag receiver on the analyzer device. The breath bag can thus not simply be used with other analyzer devices, in particular not with already existing commercial analyzer devices, which may not have an in-built breath bag receiver, but which may instead be conceived to receive an exhalation directly from the patient standing or sitting at the analyzer device. Such already existing devices do also not necessarily comprise an in-build pump system being capable of extracting the gas from the breath bag, since the breath gas flow is usually controlled by the patients themselves in many of the commercially available devices.

### SUMMARY OF THE INVENTION

In a first aspect, it is an object of the present invention to provide a storage assembly in which a gas can be safely stored without accidental leakage, while the storage assembly also enables simple and ideally sterile extraction of the gas from the storage assembly for further analysis.

This object is achieved by the storage assembly according to claim 1. Further embodiments of the inventive storage assembly are laid down in the dependent claims.

Thus, a storage assembly for storing a gas, preferably breath gas, is provided, comprising:
a bag being inflatable and configured to contain the gas;
an input piece being attached to the inflatable bag, the input piece comprising a valve mechanism having an open valve state in which a passage for the gas through the valve mechanism is open, and a closed valve state in which the passage for the gas through the valve mechanism is closed;
a sealing cap being connectable to the input piece, the sealing cap comprising a sealing membrane to seal the passage for the gas through the valve mechanism, and
a perforation device being insertable into the input piece,
wherein the perforation device comprises a perforation structure configured to perforate the sealing membrane when the sealing cap is connected to the input piece and to cause the valve mechanism to be in the open valve state once the perforation device is inserted in the input piece.

By providing a sealing cap that is connectable to the input piece, unwanted access to the valve mechanism prior and/or after filling the bag with the gas may be prevented. The sealing cap may be configured to be completely detachable from the input piece or, in order to prevent a user from dropping or losing the sealing cap, it may be configured to remain attached to the input piece in a position in which the sealing membrane does not seal the passage for the gas through the valve mechanism, for instance during filling of the bag.

Analogously, the perforation device may be configured to be completely detachable from the input piece and/or the sealing cap or to remain attached to the input piece and/or the sealing cap in order to prevent a user from dropping or losing the perforation device when it is not directly in use, in particular during filling of the bag and storage of the bag.

In addition, the storage assembly may comprise a mouthpiece which is connectable to the input piece, preferably when the sealing membrane is positioned such that it is not blocking the passage for the gas through the valve mechanism, i.e. when the passage is free, in order for a patient to exhale into the bag trough the mouthpiece. The mouthpiece may be configured to mimic a common mouthpiece as installed on commercial breath analyzer known in the art in order to provide the patient with a similar feeling during the exhalation as if the exhalation was performed directly on the breath analyzer device itself.

The bag may comprise a first sheet and a second sheet being connected, in particular glued and/or welded to each other along their edges. The first and/or the second sheet preferably comprises two or more layers, in particular preferably comprising an inner layer being in contact with the gas, wherein the inner layer may comprise or consist of polyethylene (PE), and an outer layer which may comprise or consist of polyethylene terephthalate (PET). Additionally, the first and/or the second sheet may comprise an intermediate layer being arranged between the inner layer and the outer layer, wherein the intermediate layer may comprise or consist of aluminium.

In order for the storage assembly to be particularly compact, the input piece, the sealing cap and/or the perforation device and/or the mouthpiece may be configured to be assembled along a common central axis, the common central axis being preferably in plane with the bag when the latter is empty.

The sealing cap preferably comprises a cap sleeve, the sealing membrane being preferably arranged at a first end of the cap sleeve. In particular, the cap sleeve and the sealing membrane may form a cup, the sealing membrane corresponding to a bottom of the cup.

The input piece may comprise a hollow shaft portion extending at least partially outside of the inflatable bag, wherein the hollow shaft portion is configured to at least partially receive the cap sleeve. Preferably, the input piece further comprises a connection structure to which the bag is attached in a gas-tight manner. In particular, the connection structure may be located between the first sheet and the second sheet of the bag.

The sealing cap may comprise a cap alignment feature and the input piece may comprise an input alignment feature configured to engage with the cap alignment feature to enable rotational alignment of the sealing cap with respect to the input piece.

Preferably, the valve mechanism automatically opens, i.e. moves into the open valve state, when gas is flowing through the input piece in direction of the bag, e.g. when a patient exhales through the input piece, preferably via the mouthpiece. Preferably, the valve mechanism automatically closes, i.e. moves into the closed valves state, when a pressure inside the bag exceeds a pressure outside the bag.

In order to protect the valve mechanism from damage and contaminations, the valve mechanism is preferably arranged within the hollow shaft portion of the input piece, and the sealing membrane is preferably arranged within the hollow shaft portion so as to cover and thereby seal the valve mechanism when the cap sleeve is at least partially received in the hollow shaft portion.

In preferred embodiments, the perforation device comprises an insertion portion in form of a shaft which is insertable into the cap sleeve of the sealing cap. Preferably, the cap sleeve comprises a cap guide structure and the perforation device, in particular the insertion portion, comprises a perforation guide structure configured to engage with the cap guide structure to guide the perforation device when the perforation device is inserted into the cap sleeve. The cap sleeve may be connected, in particular inserted, into to the input piece such that the sealing membrane seals the passage for the gas through the valve mechanism after filling the bag and the perforation device may subsequently be used to open the valve mechanism without having to remove the sealing cap from the input piece, thus preventing potential unwanted contamination of the valve mechanism.

The valve mechanism may be configured as a flap valve comprising a flap, the flap being kept in the open valve position by the perforation structure once the perforation device has been at least partially inserted into the input piece. Alternatively, the valve mechanism may also be configured as a duckbill valve or as an umbrella valve.

In case the valve mechanism comprises a flap, the flap may be a disk defining a disk center and a circumferential edge region. The valve mechanism may comprise a flap attachment structure, the disk being attached to the flap attachment structure at the center of the disk. To simplify fabrication, the flap may be manufacturable separately and attachable to the attachment structure in an assembly process.

Preferably, the circumferential edge region is in plane with the disk center in the closed valve position, and/or the circumferential edge region is preferably bent towards an interior of the bag in the open valve position either by a flow of gas in direction of the bag, e.g. during filling of the bag, and/or by the perforation structure, e.g. during controlled emptying of the bag, so as to allow gas to enter the bag by flowing passed the bent disk.

The flap is preferably configured to be flexible in order to be reversibly deformed, in particular bent, without being damaged, by the perforation structure of the perforation device.

The sealing membrane and/or the flap may comprise or consists of a thermoplastic elastomer (TPE) or silicone. However, silicone is less preferred, since silicone tends to produce polysiloxanes, which may cause unwanted background noise signals when the storage assembly is connected to an analyzer, in particular a mass spectrometer, for analysis of the gas contained in the bag.

The perforation structure of the perforation device is preferably arranged at a first end of the insertion portion of the perforation device. In some embodiments, the perforation structure may comprise at least two, preferably six, perforation arms, the perforation arms being preferably arranged equidistantly from each other on a circle. The flow rate of the gas through the perforated membrane generally depends on a pressure difference across the perforated membrane and a total cross section of the perforations through which the gas may flow. In practice, the flow rate of the gas exiting the bag may have to reach a predetermined value which may be defined by an analyzer device arranged downstream of the storage assembly. In order to reach said pre-determined flow rate, it may be technically easier to increase the total cross section of the perforations rather than having to increase the pressure difference. Creating a larger number of perforations with smaller diameters rather than a smaller number of perforations with larger diameters is furthermore preferred to ensure precision and reproducibility.

The perforation arms may be configured in a variety of ways. Preferably, they comprise a sharp tip portion which configured to perforate the sealing membrane without having to use excessive force. In some embodiment, each perforation arm may comprise a channel along which the gas exiting the bag may be channeled to enable an unhindered flow of the gas.

The perforation device may further comprise an orifice through which the gas exiting the bag is preferably channeled, wherein the orifice is preferably arranged at a second end of the insertion portion, the second end being opposite to the first end at which the perforation structure is preferably arranged, wherein the orifice has a diameter which may be chosen to set a desired maximum flow velocity of the gas. The orifice may have a fixed diameter, or may be configured to have an adjustable diameter.

In some embodiments, the perforation arms and the disk may be concentrically arranged, and the perforation arms may bend the circumferential edge region of the disk towards the interior of the bag once the perforation device has been at least partially inserted into the input piece.

In a second aspect, it is an object of the present invention to provide a simple and compact gauge device for determining a filling state of a bag.

This object is achieved by the gauge device according to claim 8. Further embodiments of the inventive gauge device are laid down in the dependent claims.

Thus, a gauge device for determining a filling state of a bag, preferably of the inflatable bag of the storage assembly described above, is provided, the gauge device comprising a frame structure which defines an opening into which the bag can be placed,
wherein the opening has a dimension that is adaptable to the filling state of the bag, and
wherein the gauge device comprises a filling state marker configured to indicate the dimension of the opening.

The frame structure may comprise a first frame bar delimiting the opening on a first side and a second frame bar delimiting the opening on a second side opposite to the first side. The first frame bar and the second frame bar are preferably movable with respect to each other, thereby changing the dimension of the opening.

The filling state marker may comprise a ruler part connected to the first frame bar and a cursor part connected to the second frame bar, the cursor part being configured to slide along the ruler part when the first frame bar and the second frame bar are moving with respect to each other.

Preferably, the cursor part is provided by a slit in the second frame bar and the ruler part is provided by a strip being connected in a foldable manner to the first frame bar, the strip being insertable into the slit and slidable within the slit. The strip preferably comprises at least two, in particular three ruler sections, which may correspond to different inflation states of the bag that is to be inserted into the variable opening, such as "not sufficiently inflated", "sufficiently inflated", "ideally inflated". The ruler sections may be delimited by marker lines and/or may be colored differently and/or have text written on them.

The frame structure may comprise a fold such that the frame structure is foldable from a folded state in which the second frame bar is folded onto the first frame bar and the dimension of the opening is zero, to an unfolded state in which the dimension of the opening is maximized.

The gauge device is preferably made of cardboard or a cardboard-like material that is recyclable. Alternatively, the gauge device may comprise or consist of a flexible plastic material or a metal foil.

In a third aspect, it is an object of the present invention to provide a simple method of delivering a gas sample to an analyzer assembly, wherein the method in particular does not require the analyzer assembly to contain a pump or suction system for sucking the gas into the analyzer assembly.

This object is achieved by the method according to claim 11. Further embodiments of the inventive method are laid down in the dependent claims.

Thus, a method of delivering a gas sample, preferably a breath gas sample, to an analyzer assembly, is provided, the method comprising:
providing a gas sample, preferably a breath gas sample, contained in a bag;
placing the bag in an extraction device, wherein the extraction device comprises a support structure and a squeezing structure being arranged such that the bag is received between the support structure and the squeezing structure;
establishing a gas-tight connection between the bag and the analyzer assembly, the analyzer assembly preferably comprising an ionizer device connected to a mass spectrometer;
applying pressure onto the bag via the squeezing structure to cause the gas sample to flow from the bag into the analyzer assembly.

In particular, the ionizer device may be configured as a secondary electro-spray ionization (SESI) device, which comprises a positive ionization mode ("positive mode") in which positively charged ions are generated, and a negative ionization mode ("negative mode"), in which negatively charged ions are generated.

Preferably, the gas sample is provided in the storage assembly as described above, the bag being the inflatable bag of said storage assembly, wherein the sealing cap is connected to the input piece so as to seal the valve mechanism. In such a case, the method may further comprise:
inserting the perforation device into the sealing cap;
perforating the sealing membrane with the perforation structure;
forcing the valve mechanism into the open valve state with the perforation structure to enable the gas sample to flow from the inflatable bag into the analyzer assembly.

Preferably, the perforation device is insertable into the sealing cap such that a gas-tight connection between the perforation device and the sealing cap is formed prior to perforating the sealing membrane.

Preferably, the perforation device comprises a connector part and the step of establishing a gas-tight connection between the inflatable bag and the analyzer assembly comprises:
establishing a gas-tight connection between the connector part of the perforation device and the analyzer assembly, preferably using a tube. The tube may be made of a flexible material such as polytetrafluoroethylene (PTFE). Alternatively, the tube may be a metal tube. The tube may be heated and/or may comprise an appropriate coating which may reduce a loss of the analytes present in the gas. Ideally, the tube is be kept as short as possible to minimize the loss of analytes.

The method may further comprise:
interrupting the application of pressure onto the bag after a first predetermined portion of the gas sample has been extracted from the bag;
switching a setting of the analyzer assembly, in particular in case the analyzer assembly comprises an ionizer device, switching the ionizer device between a positive ionization mode and a negative ionization mode, and
resuming the application of pressure onto the bag to cause a second predetermined portion of the gas sample to flow into the analyzer assembly.

Switching the ionizer device between a positive ionization mode and a negative ionization mode may be desirable in order to maximize the coverage of analytes which may be detected.

Preferably, the method does not comprise any steps to generate a pre-concentration of the analytes in the gas sample prior to entering the analyzer device, in particular prior to entering the ionizer device.

In a fourth aspect, it is an object of the present invention to provide a simple extraction device for controlled extraction of a gas from a bag.

This object is achieved by the extraction device according to claim 14. Further embodiments of the inventive extraction device are laid down in the dependent claims.

Thus, an extraction device for controlled extraction of a gas from a bag, preferably for use in the method described above, is provided, the extraction device comprising:
a support structure, and
a squeezing structure,
wherein the support structure and the squeezing structure are arranged such that the bag, preferably being the inflatable bag of the storage assembly as described above, can be received between the support structure and the squeezing structure,
wherein the squeezing structure is movable with respect to the support structure from a start position to an end position, and
wherein the squeezing structure is configured to exert pressure onto the bag while moving, preferably in a translatory movement, from the start position to the end position when the bag is received between the support structure and the squeezing structure to cause the gas contained in the bag to exit the bag.

In preferred embodiments, the extraction device operates entirely on mechanical principles, i.e. without any electrical parts. In particular, the translatory movement of the squeezing structure may be driven by gravity.

The extraction device may further comprise:
a stop mechanism configured to stop the squeezing structure from exerting pressure onto the bag after the squeezing structure has moved from the start position to an intermediate position located between the start position and the end position, and
a trigger mechanism configured to unlock the stop mechanism and thereby release the squeezing structure from the intermediate position.

Such a stop mechanism and trigger mechanism may be used when an interruption of the gas flow is desired, for example when the ionizer device, to which the gas is being delivered, is to be switched from the positive ionization mode to the negative ionization mode or vice versa.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows a schematic exploded view of a storage assembly according to a first embodiment of the first aspect of the present invention with disassembled components arranged along a central axis C;
- Fig. 2: shows the storage assembly of Fig. 1 in an assembled state without the perforation device and the mouthpiece shown in Fig. 1;
- Fig. 3: shows an enlarged perspective view of the input piece of Fig. 1;
- Fig. 4: shows a front view of the input piece of Fig. 3;
- Fig. 5: shows an enlarged view the flap of Fig. 1;
- Fig. 6: shows a sectional view of the input piece of Fig. 3 along a sectional plane comprising the central axis C;
- Fig. 7: shows an enlarged perspective view of the sealing cap of Fig. 1;
- Fig. 8: shows a sectional view of the sealing cap and the input piece in the assembled state of Fig. 2 along a sectional plane comprising the central axis C;
- Fig. 9: shows a perspective view the perforation device of Fig. 1;
- Fig. 10: shows a perspective view the perforation device of Fig. 9 under a different angle than in Fig. 9;
- Fig. 11: shows a sectional view of the input piece, the sealing cap and the perforation device of Fig. 1 in an assembled state along a sectional plane comprising the central axis C;
- Fig. 12: shows a schematic exploded view of a storage assembly according to a second embodiment of the first aspect of the present invention with the components arranged along a central axis C;
- Fig. 13: shows the storage assembly of Fig. 12 in an assembled state without the perforation device and the mouthpiece shown in Fig. 12;
- Fig. 14: shows an enlarged perspective view of the input piece of Fig. 12;
- Fig. 15: shows a front view of the input piece of Fig. 14;
- Fig. 16: shows an enlarged view the flap of Fig. 12;
- Fig. 17: shows a sectional view of the input piece of Fig. 14 along a sectional plane comprising the central axis C;
- Fig. 18A: shows an enlarged perspective view of the sealing cap of Fig. 12;
- Fig. 18B: shows an enlarged perspective view of the sealing cap of Fig. 12 under a different angle than in Fig. 18A;
- Fig. 19A: shows a sectional view of the sealing cap and the input piece in the assembled state of Fig. 13 along a first sectional plane comprising the central axis C;
- Fig. 19B: shows a sectional view of the sealing cap and the input piece in the assembled state of Fig. 13 along a second sectional plane comprising the central axis, the second sectional plane being perpendicular to the first sectional plane of Fig. 19A;
- Fig. 20: shows a perspective view the perforation device of Fig. 12;
- Fig. 21: shows a perspective view the perforation device of Fig. 20 under a different angle than in Fig. 20;
- Fig. 22: shows a sectional view of the input piece, the sealing cap and the perforation device of Fig. 12 in an assembled state plane along a sectional plane being parallel to and offset from the central axis C;
- Fig. 23: shows an enlarged view of an embodiment of a gauge device according to the second aspect of the present invention in an unfolded state;
- Fig. 24: shows the gauge device of Fig. 23 in a semi-folded position;
- Fig. 25: shows the gauge device of Fig. 23 together with the storage assembly of the first aspect of the present invention with the bag being in a pre-filling state;
- Fig. 26: shows the gauge device of Fig. 23 with the bag being in a filling-in-progress state;
- Fig. 27: shows the gauge device of Fig. 23 with the bag being in an ideally-filled state;
- Fig. 28: shows the storage assembly of Fig. 2 with the bag being in the ideally-filled state and sealed by the sealing cap;
- Fig. 29: shows a flow diagram of a method according to an embodiment of the third aspect of the present invention;
- Fig. 30: shows a setup for carrying out the method of Fig. 29;
- Fig. 31: shows a perspective front view of an extraction device according to a first embodiment of the fourth aspect of the present invention in a bag loading position;
- Fig. 32: shows a perspective front view of the extraction device of Fig. 31 in a start position;
- Fig. 33: shows a perspective front view of the extraction device of Fig. 31 in an intermediate position;
- Fig. 34: shows a perspective front view of the extraction device of Fig. 31 in an end position;
- Fig. 35: shows a perspective rear view of the extraction device of Fig. 31 in the end position;
- Fig. 36: shows a sectional view of the extraction device of Fig. 31 along the sectional plane E marked in Fig. 35;
- Fig. 37: shows a perspective front view of an extraction device according to a second embodiment of the fourth aspect of the present invention in a bag loading position;
- Fig. 38: shows a perspective front view of the extraction device of Fig. 37 in a start position;
- Fig. 39: shows a perspective front view of the extraction device of Fig. 37 in an intermediate position;
- Fig. 40: shows a perspective front view of the extraction device of Fig. 37 in an end position;
- Fig. 41: shows a perspective rear view of the extraction device of Fig. 37 in the end position, and
- Fig. 42: shows a sectional view of the extraction device of Fig. 31 along the sectional plane E' marked in Fig. 41.

### DESCRIPTION OF PREFERRED EMBODIMENTS

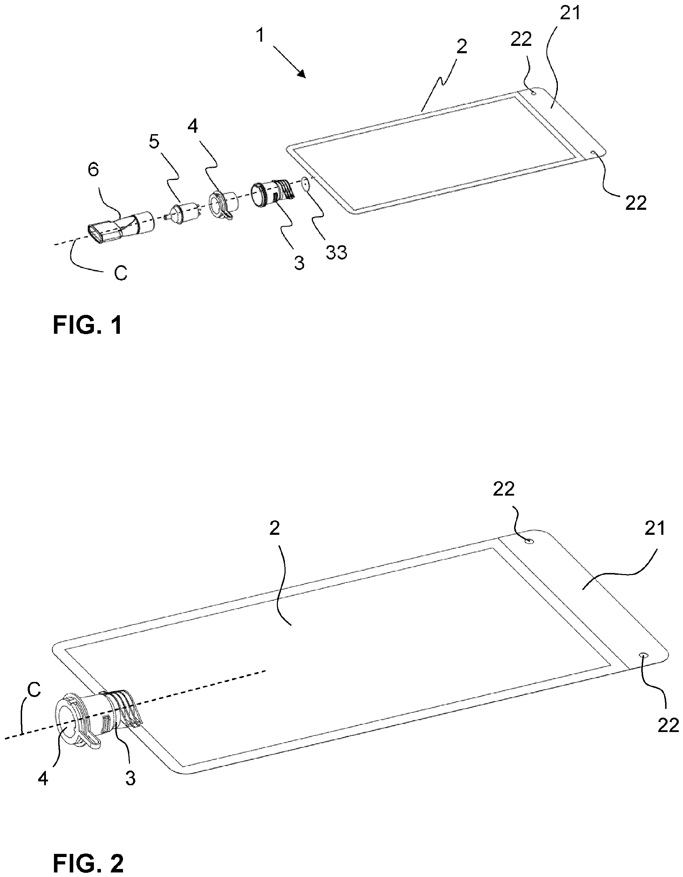

Fig. 1 shows a schematic exploded view of a storage assembly 1 for storing a gas, preferably breath gas, according to a first embodiment of the first aspect of the present invention. The storage assembly comprises an inflatable bag 2, which is configured to contain the gas, an input piece 3, a sealing cap 4, and a perforation device 5. As shown in Fig. 1, the storage assembly may further comprise a mouthpiece 6, which is connectable to the input piece 3 to enable a user to blow or exhale breath gas into the inflatable bag 2. The input piece 3, the sealing cap 4, the perforation device and the mouthpiece are configured to be assembled a long a common central axis C, as indicted in Fig. 1. The input piece 3 comprises a valve mechanism having an open valve state in which a passage for the gas through the valve mechanism is open, and a closed valve state in which the passage for the gas through the valve mechanism is closed. The sealing cap 4 is removably connectable to the input piece 3 and comprises a sealing membrane 41 to prevent gas from passing through the valve mechanism when the sealing cap 4 is connected to the input piece 3. The perforation device 5 is insertable into the input piece 3, in particular when the sealing cap 4 is in place in the input piece 3, and comprises a perforation structure 50 which is configured to perforate the sealing membrane 41 when the sealing cap is connected to the input piece 3. Furthermore, the perforation device 5 is configured to cause the valve mechanism to be in the open valve state once the perforation device 5 is inserted in the input piece 3.

Fig. 2 shows the storage assembly of Fig. 1 in an assembled state without the perforation device 5 and the mouthpiece 6. The inflatable bag 2 may be rectangular and have a fillable region with a long side having a length of 20 cm to 40 cm, preferably 30 cm and a short side having a width of 10 cm to 20 cm, preferably 14 cm. The bag 2 may be inflatable up to a maximum volume of up to 1.8 litres, preferably up to 1.4 liters or less. The inflatable bag 2 is essentially formed by a first sheet and a second sheet being connected, in particular glued and/or welded to each other along their edges.

The input piece 3 is attached to the inflatable bag 2 in the center of the short side the embodiments shown here, but may however also be located elsewhere along the edge of the bag 2 or anywhere within the fillable region away from the edge. In the embodiment shown here, the inflatable bag 2 further comprises a fixation portion 21, which is preferably a non-inflatable strip, for instance formed by a region where the first and the second sheet are glued and/or welded to each other, wherein the strip comprises fixation holes 22 for fixing/mounting/suspending the bag 2 to a corresponding bag mounting structure to hold the bag 2 in place during a filling process and/or an extraction process. In the embodiments shown here, the fixation portion 21 is arranged on a side of the filling region opposite to the input piece 3 and fixation portion 21 has two fixation holes 22. However, the fixation portion 21 may also have just one fixation hole 22 or more than two fixation holes 22. Additionally, or alternatively, the bag 2 may comprise loops and/or straps configured to fulfill the same purpose.

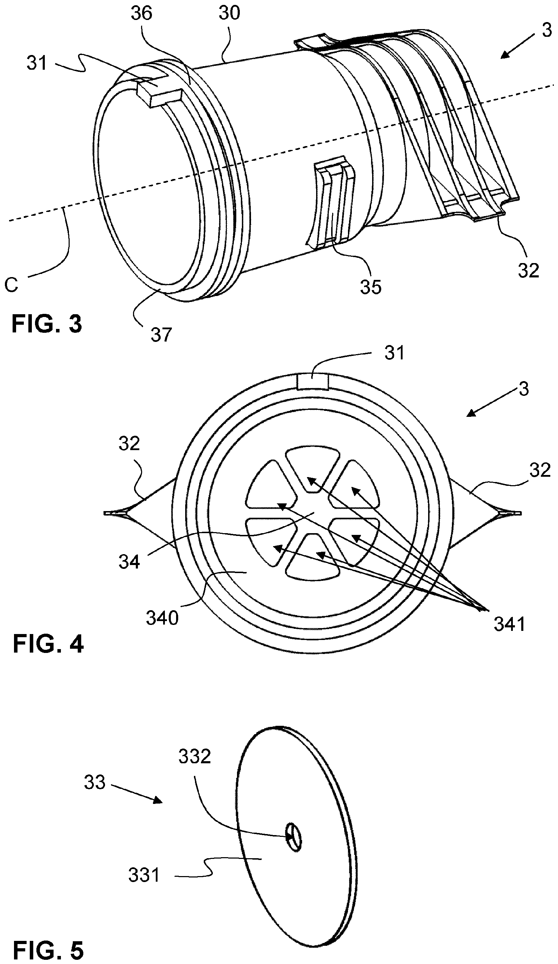

The input piece 3, which is shown separately in an enlarged perspective view in Fig. 3 and in a front view in Fig. 4, comprises a hollow shaft portion 30, which is configured to extend partially outside the inflatable bag 2, and connection structure 32, which is located between the first and the second sheet of the inflatable bag 2.

Each of the first and second sheet of the inflatable bag 2 is attached, in particular glued and/or welded and/or shrink-wrapped, to the connection structure 32 in a gas-tight manner. To facilitate the attachment of the first and second sheet of the inflatable bag 2 to the connection structure 32 of the input piece 3, the connection structure 32 comprises ridges 321 extending on two opposite sides of the hollow shaft portion 30 to form a tapered, beak-like structure on each of the two opposite sides of the hollow shaft portion 30. The first sheet is attached to a top part 322 of the beak-like structure, while the second sheet is attached to a bottom part 323 of the beak-like structure, the top part 322 and the bottom part 323 of the beak-like structure converging towards each other to form a beak tip 324 on each of the two opposite sides of the hollow shaft portion 30 of the bag connection structure 32, as visible for instance in the front view of the input piece 3 shown in Fig. 4.

The input piece 3 further comprises a mounting feature 35, which may be used to mount to input piece 3 onto a holder (not shown) during filling and/or emptying of the bag 2.

As part of the valve mechanism, the input piece 3 comprises a separation wall 340 arranged within the hollow shaft portion, the separation wall 340 having passage holes 341 through which the gas may flow in and/or out of the bag 2. Six passage holes 341 are shown in

Fig. 4, however, the number of passage holes 341 may be varied. A flap attachment structure 34 is formed at the center of the separation wall 340, the passage holes 341 being arranged in a ring-shaped manner around the flap attachment structure 34.

Fig. 5 shows a separate view of a flap 33, which is configured to be attached to the flap attachment structure 34 to form a flap valve. The flap 33 has a disk shape defining a disk center and a circumferential edge region 331. In this first embodiment, a disk hole 332 is arranged at the disk center.

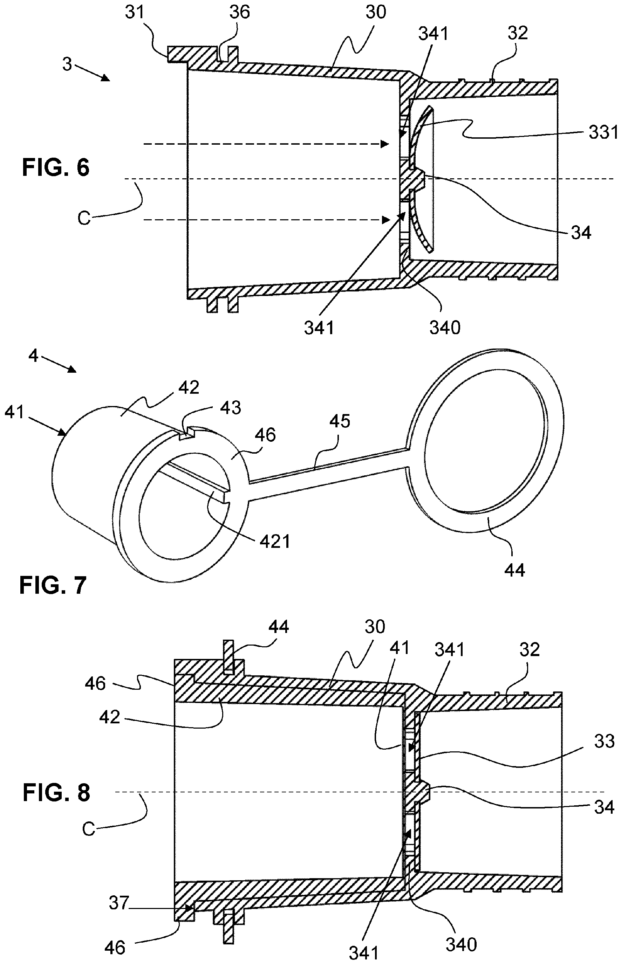

Fig. 6 shows a sectional view of the input piece 3 with the flap 33 being attached to the flap attachment structure 34, the flap attachment structure 34 in this first embodiment comprising a nub protruding through the disk hole 332 of the disk-shaped flap 33, wherein the nub comprises a circumferential notch in which the flap 33 is received. Fig. 6 shows an open valve position in which the circumferential edge region 331 of the flap 33 is bent away from the separation wall 340 towards an interior of the bag due to a gas flow passing through the passage holes 341 in direction of the bag 2, i.e. in particular coming from a patient through the mouthpiece, and hitting the flap 33 as indicated by the dashed arrows in Fig. 6.

Fig. 7 shows an enlarged perspective view of the first embodiment of the sealing cap 4 visible in Fig. 1. The sealing cap 4 comprises a cap sleeve 42 in the form of a conical cup, wherein the sealing membrane 41 is arranged at a first end of the cap sleeve 42, i.e. forming the bottom of the cup. A cap rim 46 extends radially outwards from the cap sleeve 42 at a second end of the cap sleeve 42 opposite to the first end. The cap rim 46 has an alignment feature 43 in form of a notch, which is configured to engage with the input alignment feature 31 of the input piece, which is visible in Fig. 3 and Fig. 6, to enable rotational alignment of the sealing cap 4 with respect to the input piece 3 when the sealing cap 4 is inserted into the input piece 3.

In order to secure the cap sleeve 42 to the input piece 3, i.e. for the cap sleeve 42 to remain attached to the input piece 3 when the sealing membrane 41 is not in use, the cap sleeve 42 comprises a retaining feature 44. In this first embodiment, the retaining feature 44 has the form of a retaining ring, the retaining feature 44 being connected to the cap rim 46 via a retaining feature connection part 45 in form of a bendable strip. In this first embodiment, the input piece 3 has a retaining notch 36 running circumferentially along an outer surface of the hollow shaft portion 30 to receive the retaining feature 44, i.e. the retaining ring.

Fig. 8 shows a sectional view of the sealing cap 4 being inserted in the hollow shaft portion 30 of the input piece 3, wherein the cap rim 46 abuts on an abutment edge 37 of the hollow input shaft 30. The retaining ring 44 is received in the retaining notch 36 of the input piece 3. In this inserted state, the bendable strip 45 is bent in a U-shape such that the cap sleeve 42 is arranged concentrically within the retaining ring 44, as visible for instance in Fig. 2. As shown in Fig. 8, in this first embodiment, the sealing membrane 41 abuts on the separation wall 340, i.e. is in direct contact with the latter, and thereby covers the passage holes 341 to seal the valve mechanism. In Fig. 8, the valve mechanism is shown in a closed valve state, i.e. the circumferential edge region 331 of the flap 33 is in plane with the disk center, wherein the circumferential edge region 331 of the flap 33 extends radially beyond the passage holes 341 and rests on the separation wall 340, thus covering the passage holes 341 from a side of the separation wall 340 that is opposite to the side of the separation wall that is configured to be covered by the sealing membrane 41. In this closed valve state, the gas is trapped inside the inflatable bag 2. Increasing a pressure of the gas inside the bag 2 leads to an increased pressure against the circumferential portion 331 of the flap 33, thus causing the latter to be pressed more strongly against the separation wall 340. The valve mechanism thus acts as a passive one-way valve, wherein the gas cannot exit the bag unless the flap 33 is forced open by external means, e.g. by the perforation device 5.

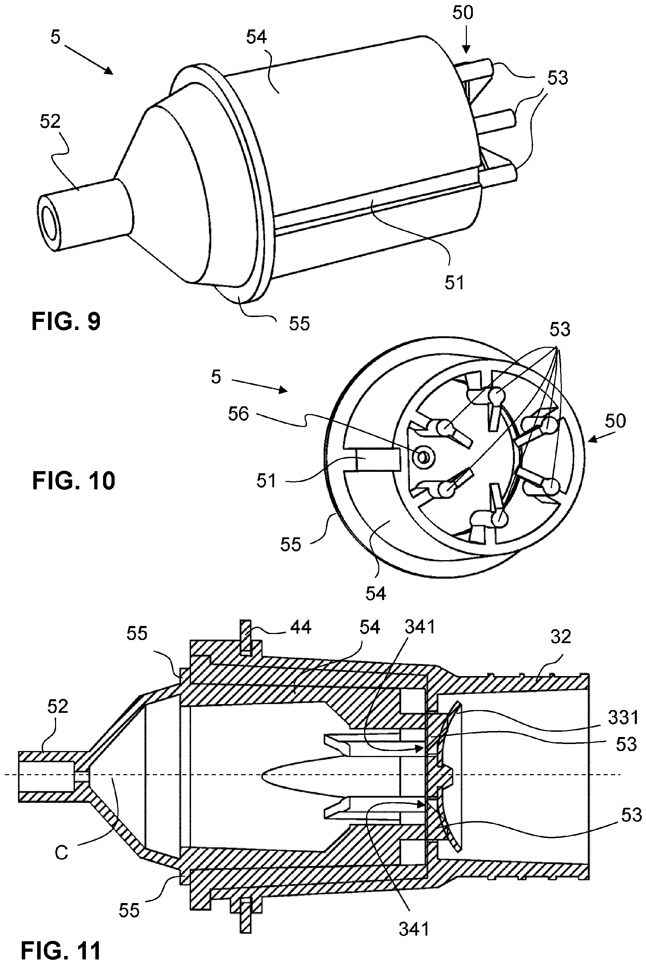

Fig. 9 shows a separate perspective view of the first embodiment of the perforation device 5. Fig. 10 shows the same embodiment of the perforation device 5 under a different viewing angle than in Fig. 9. The perforation device 5 comprises an insertion portion 54 in form of a shaft which is insertable into the cap sleeve 42 of the sealing cap 4. A perforation structure 50 is arranged at a first end of the insertion portion 54, the perforation structure 50 being configured to perforate the sealing membrane 41 and to cause the valve mechanism to be in the open valve state once the perforation device 5 is inserted in the input piece 3 when the sealing cap 4 is connected to the input piece 3. In this first embodiment, the perforation structure 50 comprises six perforation arms 53 which are arranged equidistantly from each other on a circle. Each perforation arm 53 comprises a triangular tip pointing radially inwards towards a center of the circle. An abutment ring 55 is arranged at a second end of the insertion portion 54 opposite to the perforation structure 50, the abutment ring being configured to abut on the cap rim 46 of the sealing cap 4 when the perforation device is inserted in the sealing cap 4, thus defining a maximum insertion depth. To guide the insertion, the insertion portion 54 comprises perforation guide structure 51 in the form of a groove running axially from the first end to the second end of the insertion portion 54, the groove being configured to engage with a cap guide structure 421, which in this first embodiment is a ridge formed on an inner surface of the cap sleeve 42, as shown in Fig. 7.

As depicted in a sectional view of the input piece 3, the sealing cap 4 and the perforation device 5 in Fig. 11, the perforation arms 53 are configured to punch trough the sealing membrane 41 and to subsequently bend the circumferential edge region 331 of the flap 33 towards the interior of the bag 2, so as uncover the passage holes 341 and thus enable the gas to exit the bag 2 through the passage holes 341 and the perforated sealing membrane 41.

The perforation device 5 comprises an orifice 56 arranged at the second end of the insertion portion 54, the orifice 56 having a diameter which may be chosen to set a desired maximum flow velocity of the gas. The orifice 56 may have fixed diameter, as shown in Fig. 10, or may be configured to have an adjustable diameter, in a manner similar to a shutter of a camera, in other embodiments (not shown). The perforation device 5 further comprises a connector part 52, preferably arranged downstream of the orifice 56 as shown in Figs. 9 and 11, at the second end of the insertion portion 54. The connector part 52 is preferably configured to be connectable, either directly or via a connecting tube, to an analyzer assembly to analyze the gas exiting the bag 2. For this purpose, the connector part 52 may comprise a hollow shaft into which a tube may be inserted. Preferably, the input piece 3, the sealing cap 4 and the perforation device are configured to form a gas-tight connection when properly assembled, i.e. such that the gas can only exit through the connector part 52 of the insertion device 5.

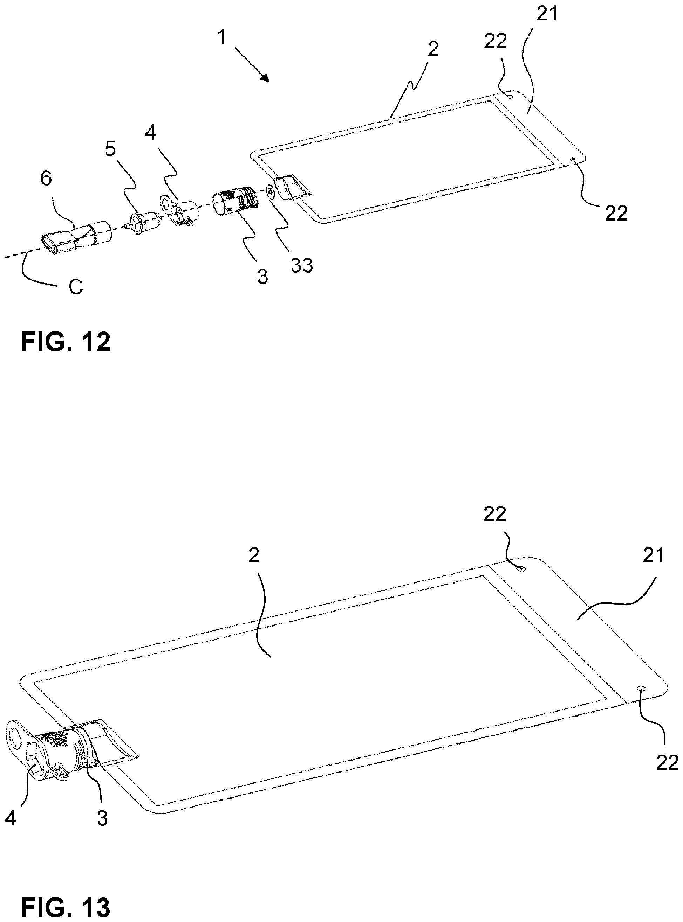

Figs. 12-22 shows a storage assembly 1 according to a second embodiment of the first aspect of the present invention, the detailed views of Figs. 12-22 being arranged in the same order as the views shown for the first embodiment in Figs. 1-11. Features that fulfill a similar function as in the first embodiment are marked in Figs. 12-22 with the same reference signs as in Figs. 1-11. The description of Figs. 1-11 applies essentially to the second embodiment of the storage assembly as well. In the following, only the difference between the two embodiments will be highlighted.

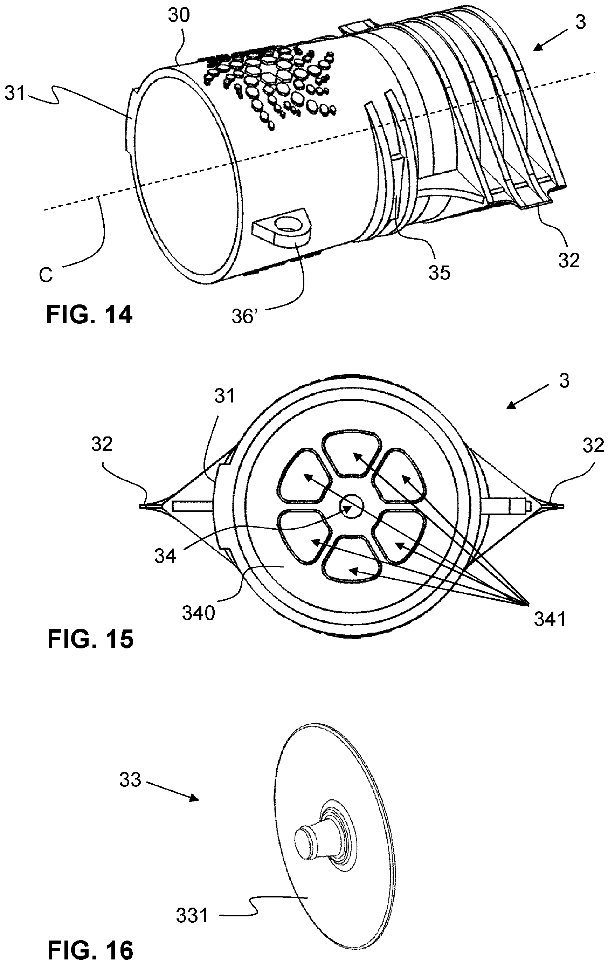

As shown in Figs. 14 and 15, the input alignment feature 31 of the second embodiment protrudes radially from the hollow shaft portion 30 with respect to the central axis C, while it protrudes axially in the first embodiment shown in Fig. 3.

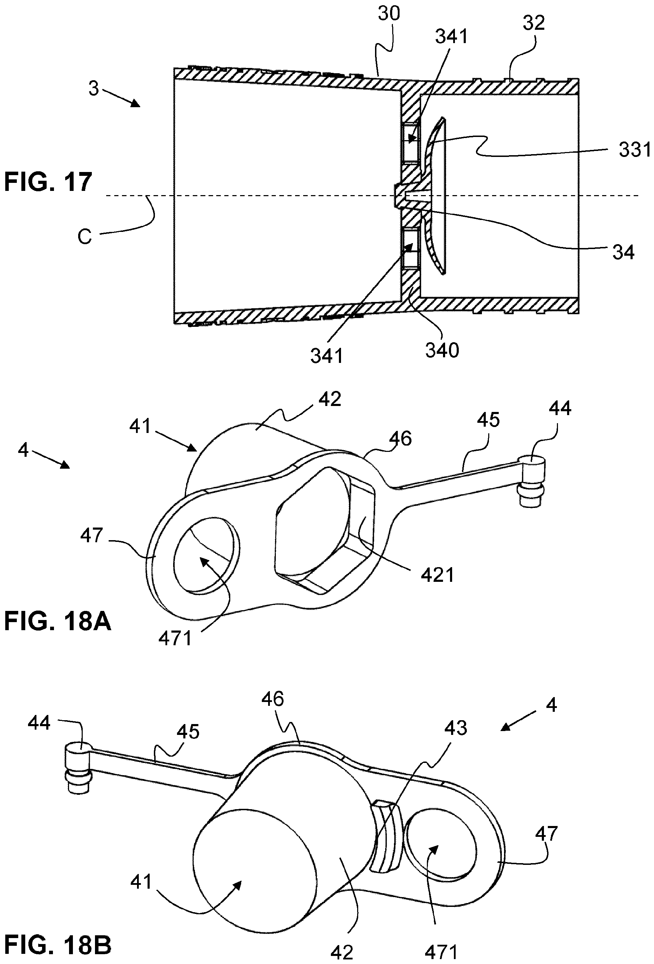

As shown in Fig. 15, the flap attachment structure 34 in the second embodiment is configured as a center hole in the separation wall 340, wherein the disk-shaped flap 33 of the second embodiment, shown separately in Fig. 16, comprises a flap nub perpendicularly extending from the center of the disk-shaped flap 33. In an assembled state, the flap nub is received in the center hole of the separation wall 340, as visible in the sectional view of the second embodiment of the input piece 3 shown in Fig. 17.

The sealing cap 4 according to the second embodiment, shown separately in Figs. 18A and 18B, comprises a cap retaining feature 44 that is shaped as a plug instead of a ring as in the first embodiment shown in Fig. 7. The plug is insertable into a retaining plug hole 36', which is arranged in a ledge extending from an outer surface of the hollow shaft portion 30 of the second embodiment of the input piece 3, as shown in Figs. 13 and 14. In order to facilitate handling of the sealing cap 4, the sealing cap 4 according to the second embodiment comprises a handle portion 47, which is formed in once piece with the cap rim 46 and comprises a grip hole 471 configured to be gripped by a human finger.

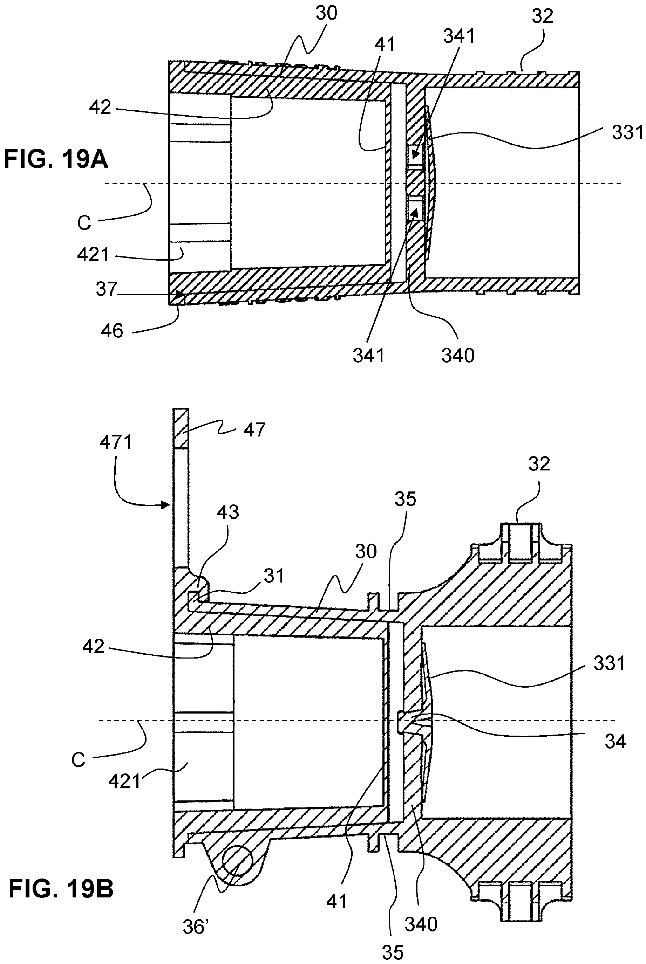

Fig. 19A shows a first sectional view of the sealing cap 4 being inserted in the hollow shaft portion 30 of the input piece 3, wherein the cap rim 46 abuts on an abutment edge 37 of the hollow input shaft 30. In this second embodiment, the sealing membrane 41 is not in direct contact with the separation wall 340, i.e. the sealing membrane 41 is arranged at a distance from the separation wall 340 when the sealing cap 4 is maximally inserted in the input piece 3.

Fig. 19B shows a second sectional view of the sealing cap 4 being inserted in the hollow shaft portion 30 of the input piece 3 along a different sectional plane than in Fig. 19A, the sectional planes of Fig. 19A and Fig. 19B being orthogonal to each other. In both Fig. 19A and Fig. 19B, the valve mechanism is shown in a closed state. Fig. 19B shows the cap alignment feature 43 which, in contrast to the first embodiment, is arranged on a rear side of the rim 46, i.e. facing towards the first end of the cap sleeve 42 at which the sealing membrane 41 is arranged. The cap alignment feature 43 is visible in the view shown in Fig. 18B. In this second embodiment, the cap alignment feature 43 comprises a rib arranged adjacent to the cap sleeve 42 so as to form a slit-shaped receiving portion configured to receive the input alignment feature 31 (also visible in Figs. 14 and 15), wherein the cap sleeve 42 is first inserted into the hollow shaft 30 of the input piece 3 and then pushed and/or rotated with respect to the input piece 3 until the input alignment feature 31 snaps into the slit-shaped receiving portion.

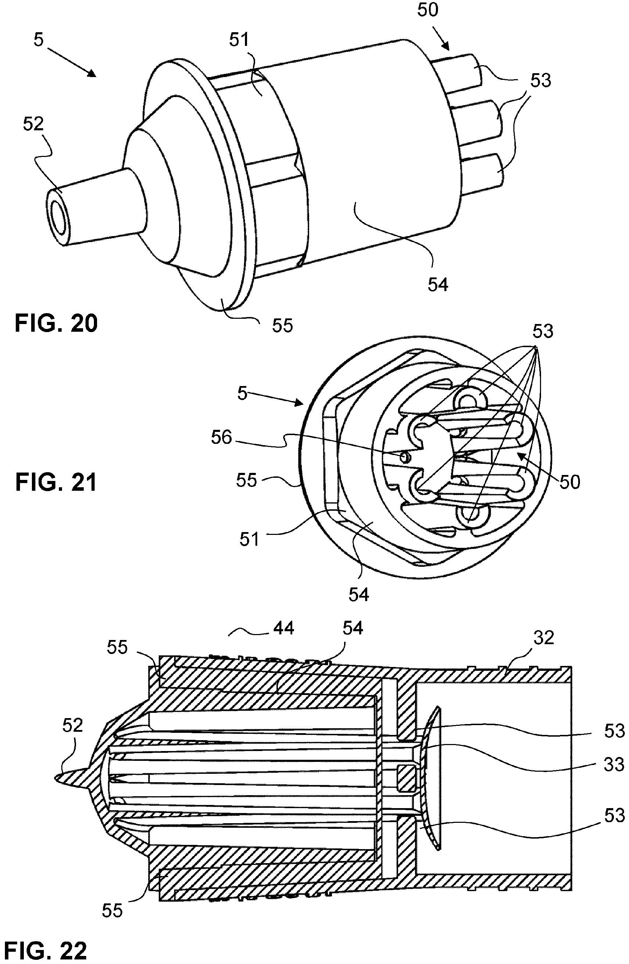

The cap guide structure 421, which engages with the perforation guide structure 51 of the perforation device 5, also differs from the first embodiment: in the second embodiment, the cap guide structure 421 is formed by a guide section of the cap sleeve 42 having a noncircular cross section, in particular a hexagonal cross section, the guide section being arranged at the second end of the cap sleeve 42, i.e. opposite to the first end at which the sealing membrane 41 is arranged, as shown in Fig. 18A. The perforation device 5 of the second embodiment, shown separately under two different angles in Figs. 20 and 21, comprises a sub-portion of the insertion portion 54 that acts as the perforation guide structure 51, the sub-portion 51 being shaped to fit into the guide section 421 of the cap sleeve 42 when properly oriented with respect to the cap sleeve. In particular, as shown in Figs. 20 and 21, the sub-portion 51 has a hexagonal cross section as well if the cap guide structure 421 has a hexagonal cross section. In contrast to the perforation device 5 of the first embodiment, the perforation device 5 of this second embodiment shown in Figs. 20 and 21 may be easier to align with respect to the cap sleeve 42, since it may be inserted into the cap sleeve 42 under six different rotation angles, as compared to having only one possible angular orientation defined by the engagement of the groove and the ridge in the first embodiment (see Figs. 7 and 9).

The perforation structure 50 of the second embodiment of the perforation device 5 also comprises six perforation arms 53 which are arranged equidistantly from each other on a circle. However, the perforation arms 53, visible in Figs. 21, are shaped differently than the ones of the first embodiment shown in Fig. 10. In this second embodiment, the perforation arm 53 are hollow partial cylinders extending parallel to the central axis and being open towards a center of the circle on which they are arranged. Each of the hollow partial cylinders has an edge in the form of a circle segment, which is sharp enough to perforate the sealing membrane 41. The perforation arms 53 of this second embodiment are forming channels along which gas exiting the bag 2 may be guided towards the orifice 56 before entering the connector part 52 of the perforation device 5.

Fig. 22 shows a sectional view of the input piece 3, the sealing cap 4 and the perforation device 5 of the second embodiment being assembled, i.e. wherein the perforation arms 53 are reaching through the ruptured sealing membrane 41 and forcing the circumferential edge region 331 of the flap 33 to uncover the passage holes 341 by bending the circumferential edge region 331 of the flap 3 towards the interior of the bag 2. Note that the sectional plane in Fig. 22 is parallel to the central axis C, but arranged at a distance from the central axis C, in contrast to the sectional view of the first embodiment shown in Fig. 11, where the sectional plane comprises the central axis C. Thus, only a small cut of the connector part 52, which is conically tapered in this second embodiment, is visible in the sectional view of Fig. 22.

The features of each component of the first and the second embodiment of the storage assembly 1 shown in Figs. 1-11 and Figs 12-22, respectively, may of course be interchanged and mixed to create a variety of additional embodiments not explicitly shown in the drawings.

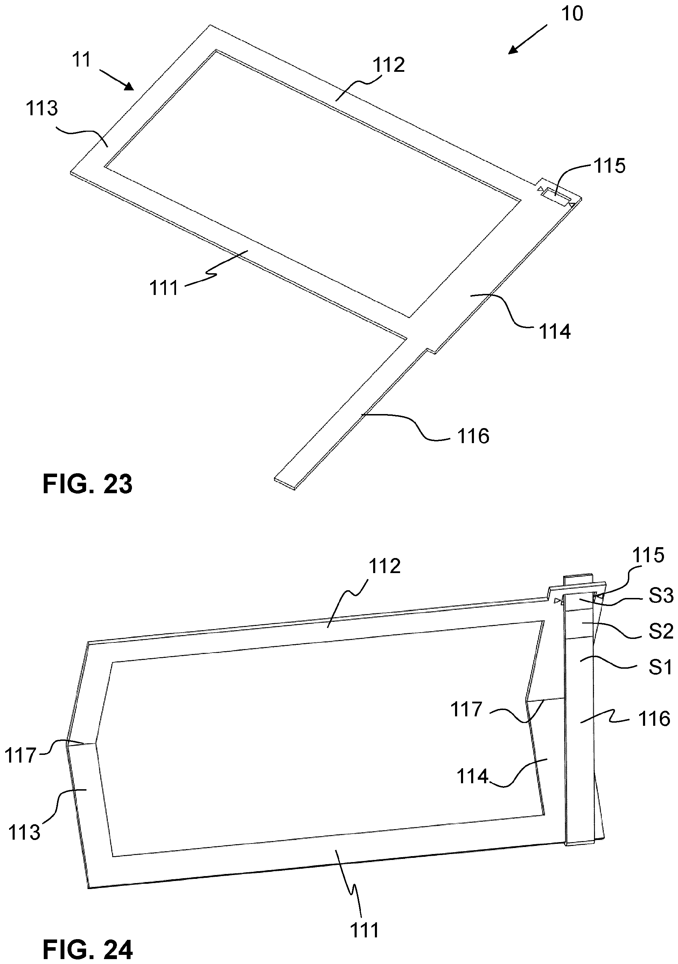

Figs. 23 and 24 show an enlarged view of a gauge device 1 according to the second aspect of the present invention. The gauge device 10 comprises a frame structure 11, which defines an opening into which a bag, in particular the inflatable bag 2 of storage assembly 1 according to the first aspect of the present invention, can be placed. The opening has a dimension that is adaptable to the filling state of the bag, in particular the inflation state of the inflatable bag 2. The frame structure 11 comprises a first frame bar 111 delimiting the opening on a first side and a second frame bar 112 delimiting the opening on a second side opposite to the first side. The first frame bar 111 and the second frame bar 112 are movable with respect to each other, thereby changing the dimension of the opening. In the present embodiment, the first frame bar 111 and the second frame bar 112 are parallel to each other and are connected by a third frame bar 113 and a fourth frame bar 114, the third frame bar 113 and the fourth frame bar 114 extending perpendicularly to the first frame bar 111 and the second frame bar 112 so as to form a rectangle when the frame structure 11 is in an unfolded state, as shown in Fig. 23, in which all frame bars 111,112,113,114 lie in a common plane and the dimension of the opening is at its maximum. The frame structure 11 comprises a fold 117, the fold 117 extending across the third frame bar 113 and the third frame bar 114 in a line that is parallel to the first 111 and the second 112 frame bar, and that is arranged equidistantly from the first 111 and the second 112 frame bar, such that the first frame bar 111 can be folded onto the second frame bar 112 to a folded state, in which the dimension of the opening is then reduced to zero.

The gauge device 10 further comprises a filling state marker configured to indicate the dimension of the opening, the filling state marker in the present embodiment comprising a ruler part in the form of a strip 116 and a cursor part in the form of a slit 115 in the second frame 112 bar, into which the strip 116 can be inserted. In the present embodiment, the strip 116 extends perpendicularly away from the first frame bar 111 in the unfolded state so as to be in-line with the third frame bar 113, and the slit is arranged in a corner of the frame structure 11 shared by the second frame bar 112 and the third frame bar 113. The strip 116 is connected to the first frame bar 111 in a foldable manner, i.e. by a fold at an outer edge of the first frame bar 111, such that the strip can be folded back onto the third frame bar 113. In order to be inserted into the slit 115, the frame structure 11 may be folded into a semi-folded position as shown in Fig. 24. The strip comprises three ruler sections S1,S2,S3, which correspond to different inflation states of the bag 2 that is to be inserted into the variable opening, such as "not sufficiently inflated" S1, "sufficiently inflated" S2, "ideally inflated" S3. The ruler sections S1,S2,S3 may be delimited by marker lines and/or may be colored differently and/or have text written on them. The cursor, i.e. the slit 115, is located in the different ruler section depending on the dimension of opening, i.e. here the distance between the first frame bar 111 and the second frame bar 112, thereby providing an indicating about the current dimension of the opening. In order for the strip 116 to easily slide through slit 115 without getting stuck, the slit 115 may have a width that is larger than a thickness of the strip 116 to allow some mechanical play. To provide a reliable indication of the current dimension of the opening, in the embodiment shown here, the center of the slit 115 is marked by two arrows, i.e. one arrow being arranged on either side of the slit 115.

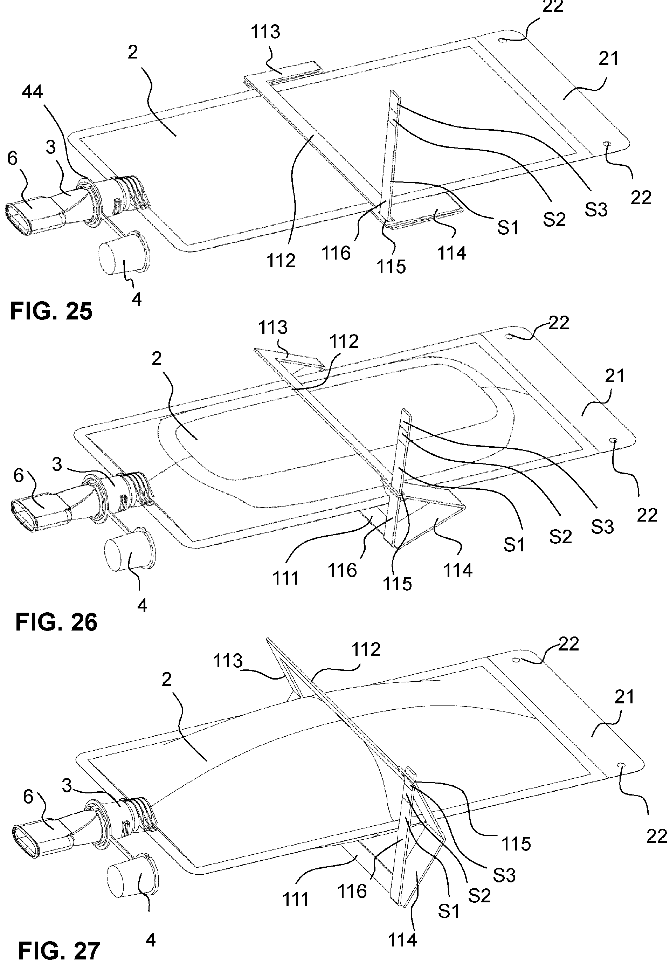

Figs. 25-27 show the working principle of the gauge device 10 to determine the inflation state of the inflatable bag 2 of the first embodiment of the storage assembly 1 shown in Figs. 1-11. The gauge device 10 may however be used in an equal manner for determining the inflation state of the inflatable bag 2 of the second embodiment of the storage assembly 1 shown in Figs. 12-22.

Fig. 25 depicts a pre-filling state, in which the inflatable bag 2 is empty and in which the cap sleeve 42 of the sealing cap 4 is not inserted in the input piece 3, but wherein the sealing cap 4 is nevertheless attached to the input piece 3 by the retaining feature 44, and wherein the mouthpiece 6 has been inserted into the input piece 3 to enable a user to exhale into the inflatable bag 2. The frame structure 11 of the gauge device 10 surrounds the bag 2, which is flat in the pre-filling state, such that the frame structure 11 is essentially in the folded state, with the strip 116 extending vertically upwards through the slit 115.

Fig. 26 depicts a filling-in-progress state, in which the bag 2 is partially filled, i.e. partially inflated. As the bag 2 inflates, it is pushing the first frame bar 111 and the second frame bar 112 away from each other, which causes the ruler part, i.e. the strip 116, to slide with respect to the cursor part, i.e. the slit 115. In Fig. 15, the slit 115 is still located in the first ruling section S1 indicating that the bag 2 is not yet sufficiently inflated.

Fig. 27 depicts an ideally-filled state, in which the inflatable bag 2 has been inflated to an ideal volume. The slit 115 is located in the third ruler section S3, which indicates that the bag 2 is ideally inflated.

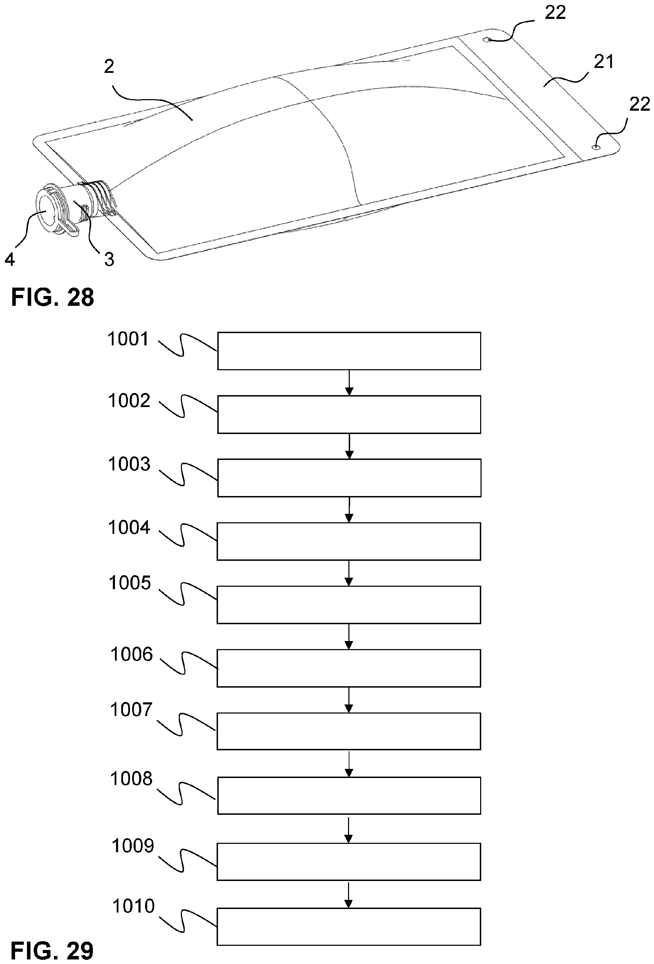

After having been inflated, the bag 2 may be sealed by inserting the cap sleeve 42 of the sealing cap 4 into the input piece 3, as shown in Fig. 28. In this sealed state, the bag may be transported or shipped to an analyzer assembly for performing an analysis of the gas contained in the bag 2.

In Fig. 29, a method of delivering a gas sample contained in a bag to an analyzer assembly according to an embodiment of the third aspect of the present invention, is schematically represented as a flow diagram.

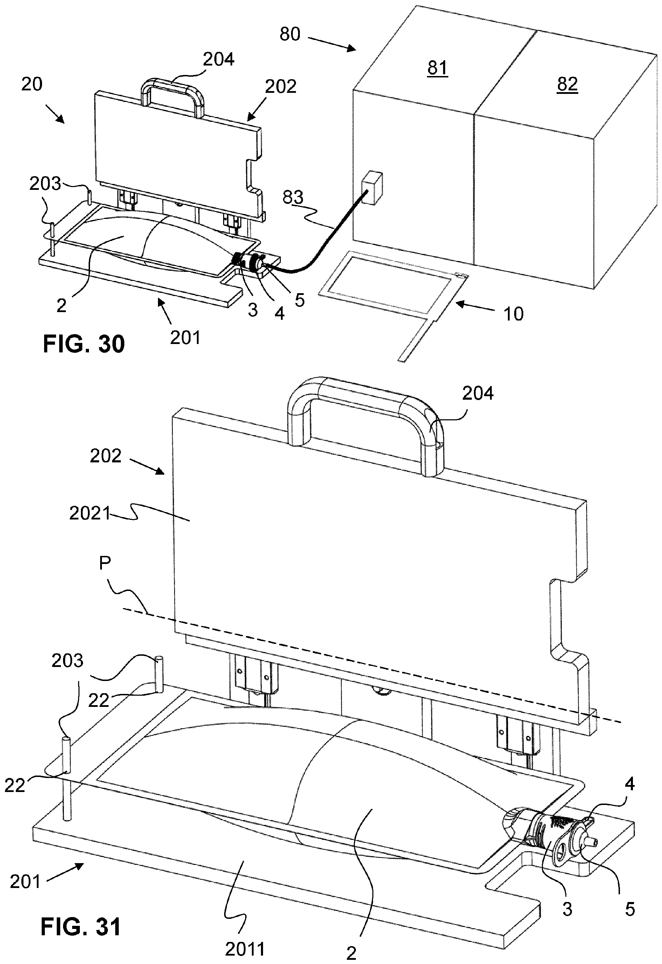

Fig. 30 shows a setup in which said method may be performed, the setup comprising an extraction device 20 to extract the gas from the bag 2 in a controlled manner according to an embodiment of the fourth aspect of the present invention, and the analyzer assembly 80. The analyzer assembly in this example comprises an ionizer device 81 that is connected to a mass spectrometer 82. Preferably, the ionizer device 81 is configured as a secondary electro-spray ionization (SESI) device, which comprises a positive ionization mode ("positive mode"), and a negative ionization mode ("negative mode").

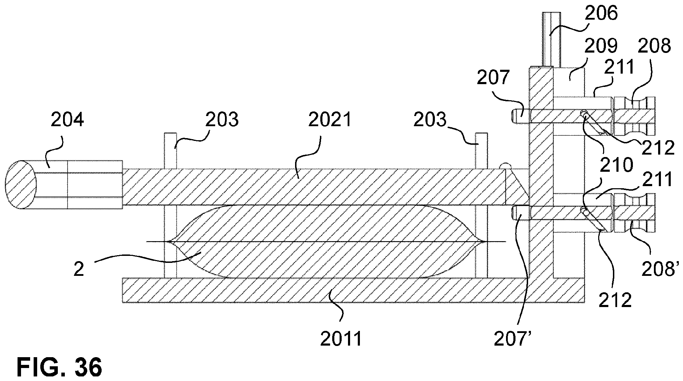

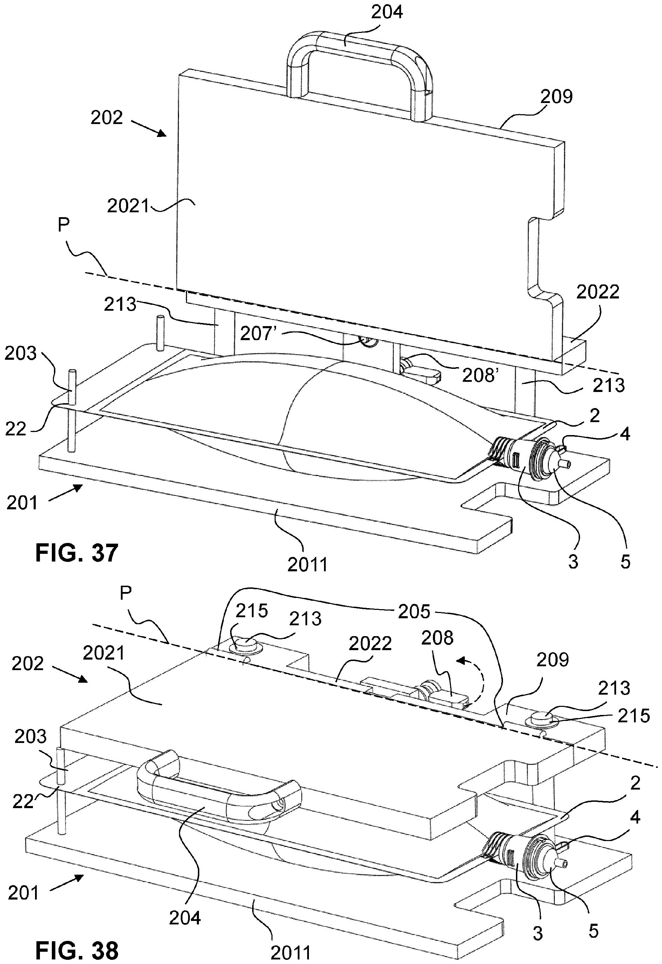

A first embodiment of the extraction device 20 is shown in enlarged perspective views in Figs. 31-35 and in a sectional view in Fig. 36. A second embodiment of the extraction device 20 is shown in Figs. 37-42.

In the figures, the first embodiment of the extraction device 20 is shown together with the second embodiment of the storage assembly 1, while the second embodiment of the extraction device 20 is shown together with the first embodiment of the of the storage assembly 1. However, both the first and the second embodiment of the extraction device 20 can of course be used with either of the first and second embodiment of the storage assembly 1 shown in Figs. 1-22, or with any other storage assembly that comprises a flexible bag.

The extraction device 20 comprises a support structure 201 and a squeezing structure 202, wherein the support structure 201 and the squeezing structure 202 are arranged such that the bag 2 can be received between the support structure 201 and the squeezing structure 202. The squeezing structure is movable with respect to the support structure 201 from a start position to an end position and the squeezing structure is configured to exert pressure onto the bag 2 while moving from the start position to the end position to cause the gas sample contained in the bag 2 to exit the inflatable bag 2.

A preferred embodiment of the method, in which a breath gas sample is provided in the storage assembly of any of the embodiments described above, comprises the following steps schematically depicted in Fig. 29:
1001 providing the breath gas sample in the inflatable bag 2;
1002 placing the inflatable bag 2 in the extraction device 20 such that the inflatable bag is received between the support structure 201 and the squeezing structure of the extraction device 20;
1003 establishing a gas-tight connection between the inflatable bag 2 and the analyzer assembly 80, wherein the gas-tight connection is established between the connector part 52 of the perforation device 5, in particular using a tube 83, wherein the tube 83 may be a metal tube or a tube made of a flexible material;
1004 inserting the perforation device 5 into the sealing cap 4;
1005 perforating the sealing membrane 41 with the perforation structure 50;
1006 forcing the valve mechanism into the open valve state with the perforation structure 50 to enable the gas sample to flow from the inflatable bag 2 into the analyzer assembly 80, wherein the perforation device 5 is insertable into the sealing cap 4 so as to form a gas-tight connection between the perforation device 5 and the sealing cap 4 prior to perforating the sealing membrane 41;
1007 applying pressure onto the inflatable bag 2 via the squeezing structure 202 to cause the gas sample to flow from the bag 2 into the ionizer device 81;
1008 interrupting the application of pressure onto the inflatable bag 2 after a first predetermined portion of the gas sample has been extracted from the inflatable bag 2;
1009 switching the ionizer device 81 between a positive ionization mode and a negative ionization mode, and
1010 resuming the application of pressure onto the inflatable bag 2 to cause a second predetermined portion of the gas sample to flow into the ionizer device 81.

Depending on the desired measurement, the switching step 1009 may also be omitted. Furthermore, pressure may be applied to the inflatable bag 2 already prior to opening the valve mechanism, i.e. prior to perforating the sealing membrane 41. In addition, the method may further comprise heating the gas-tight connection between the inflatable bag 2 and the analyzer assembly, in particular heating the tube 83.

Fig. 31 and Fig. 37 show the first and the second embodiment, respectively, of the extraction device 20 in a bag loading position. In the bag loading position, the squeezing structure 202 is positioned such that the bag 2 can conveniently be placed onto the support structure 201. As shown in both embodiments, the squeezing structure 202 comprises a squeezing plate 2021, the squeezing plate 2021 being substantially rectangular except for a cut-out to accommodate the input piece 3 of the storage assembly 1, and a handle 204 attached to a first edge of the squeezing plate 2021, as well as a hinge mechanism 205 arranged on a second edge of the squeezing plate 2021 being opposite to the first edge. The support structure 201 comprises a support plate 2011, and the hinge mechanism 205 causes the squeezing plate 2021 to be pivotable about a pivot axis P which lies in a plane that is parallel to the support plate 2011.

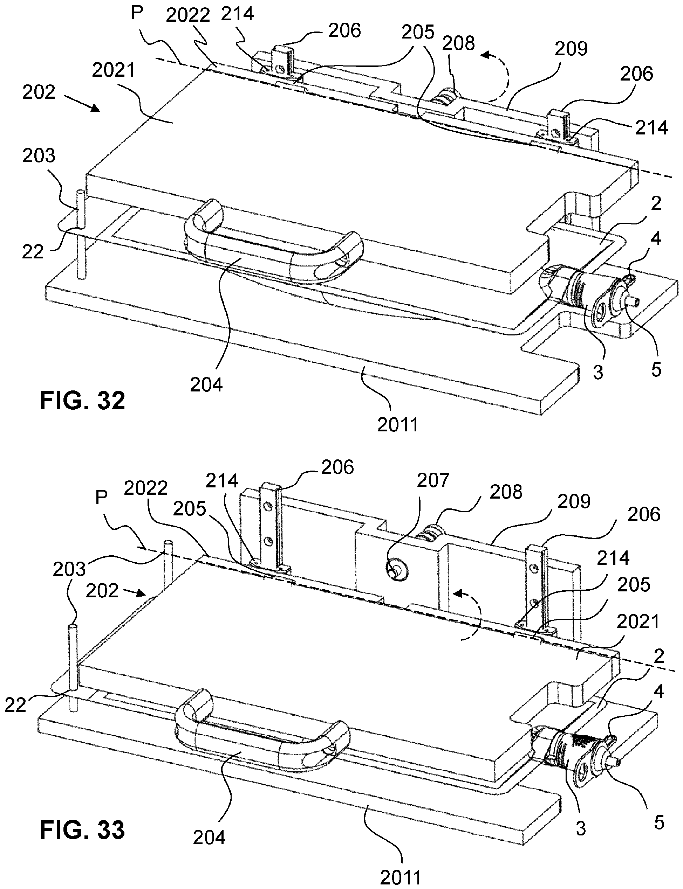

In particular, the squeezing plate 2021 may be pivoted by 90° about the pivot axis P from the bag loading position, in which the squeezing plate 2021 does not touch the bag 2, to a start position, which is depicted in Fig. 32 and Fig. 38, in which the squeezing plate 202 and the support plate 2011 are parallel to each other.

In order for the inflatable bag 2 to hold in place between the support plate 201 and the squeezing plate 202, two mounting posts 203 are attached to the support plate 2011 so as to extend vertically from the support plate 2011, wherein the mounting posts 203 are separated by a distance that corresponds to a distance between the fixation holes 22 of the fixation portion of the bag 2. The bag 2 is then arranged such that each of the mounting posts 203 extends through one of the fixation holes 22 to prevent the bag 2 from moving off the support plate 2011. Alternatively, in an embodiment not shown here, a single mounting post 203 may also be used instead, in particular if the fixation portion of the bag 2 only has one single fixation hole, the fixation hole being preferably arranged in the center of the fixation portion, and the single mounting post 203 being arranged accordingly on the support plate 2011.

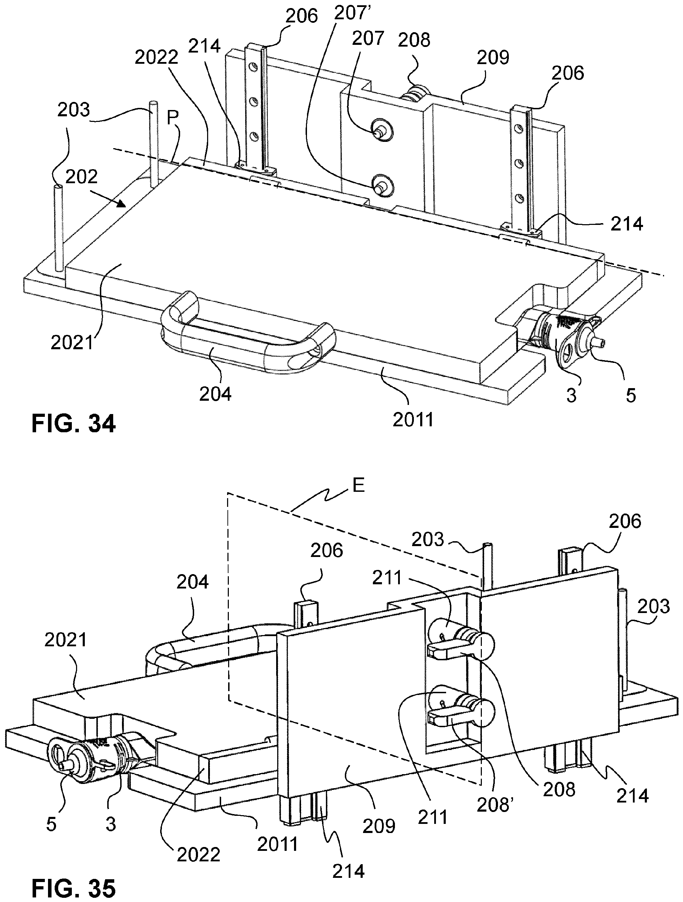

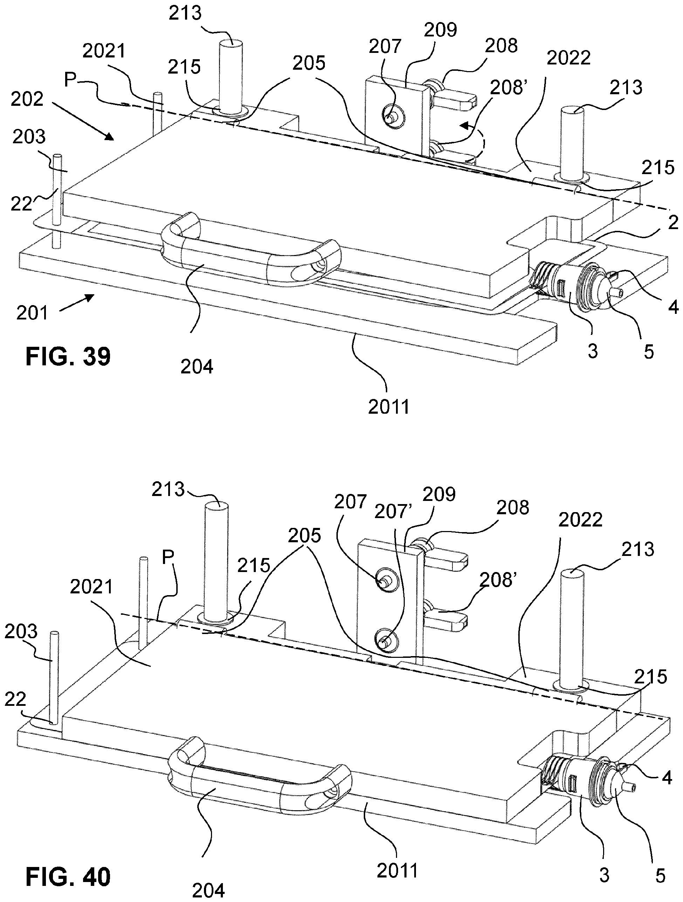

In both embodiments, the squeezing plate 202 is movable from the start position to an intermediate position, which is depicted in Fig. 33 and Fig. 39, in which the bag 2 is partially deflated, i.e. in which a first predetermined portion of the gas sample has been extracted from the bag 2 and a second predetermined portion of the gas sample is still in the bag 2. The squeezing plate 202 of both embodiments of the extraction device 20 is further movable from the intermediate position to an end position, which is shown in Fig. 34 and Fig. 40. In the end position shown here, the bag 2 is preferably maximally deflated, i.e. preferably all the gas has been extracted from the bag 2. In other embodiments not shown here, it may however be conceivable for the end position to correspond to a state in which the bag 2 is not yet empty.

In both embodiments, the squeezing plate 202 moves from the start position to the intermediate position and further from the intermediate position to the end position by sliding along a guide system.

In the first embodiment shown in Figs. 31-36, the guide system comprises two rails 206. The two rails 206 run parallel to each other and are attached at a distance from each other to a wall structure 209, wherein the wall structure 209 is fixedly arranged with respect to the support plate 2011 and extends vertically from the latter. The guide system further comprises two slide carriages 214, wherein each slide carriage 214 is guided on one of the two rails 206. The squeezing structure 202 comprises a connection portion 2022, to which the squeezing plate 2021 is connected via the hinge mechanism 205, the connection portion 2022 being attached to the two slide carriages 214 so as to be vertically movable along the rails 206 in order to enable the squeezing plate 2021 to move up and down.

In the second embodiment shown in Figs. 37-42, the guide system comprises two slide posts 213 extending vertically from the support plate 2011 and fixed at a distance from each other on the support plate 2011. The connection portion 2022 of the second embodiment comprises two slide holes, wherein each slide hole 215 is associated with one of the slide posts 213 such that each slide post 213 extends through one of the slide holes 215, thereby forming a gliding mechanism with a friction that is sufficiently low for the connection portion 2022 to be able to glide up and down the slide posts 213, thus enabling the squeezing plate 2021, which is attached to the connection portion 2022 via the hinge mechanism 205, to move up and down. The second embodiment of the extraction device 20 also comprises a wall structure 209 being fixedly arranged with respect to the support plate 2011 and extending vertically from the latter. However, wall structure 209 is arranged between the slide posts 213 and is not in direct contact with the slide posts 213.

In the embodiments of the extraction device shown in Figs. 30-42, the movement of the squeezing structure 201 from the start position to the intermediate position and from the intermediate position to the end position is caused by gravity, i.e. the pressure applied to the bag 2 by the squeezing structure 202 while moving is determined by the weight of the squeezing structure 202 per area.

In order to stop the squeezing structure 202 from exerting pressure onto the bag 2 after the squeezing structure 202 has moved from the start position to an intermediate position located between the start position and the end position, the extraction device 20 comprises a stop mechanism. The extraction device 20 further also comprises a trigger mechanism configured to unlock the stop mechanism and thereby release the squeezing structure 202 from the intermediate position. In the embodiments of the extraction device shown in Figs. 30-42, the stop mechanism is further configured to keep the squeezing structure 202 in the start position and the trigger mechanism is further configured to release the squeezing structure 202 from the start position to allow it to move from the start position to the intermediate position.

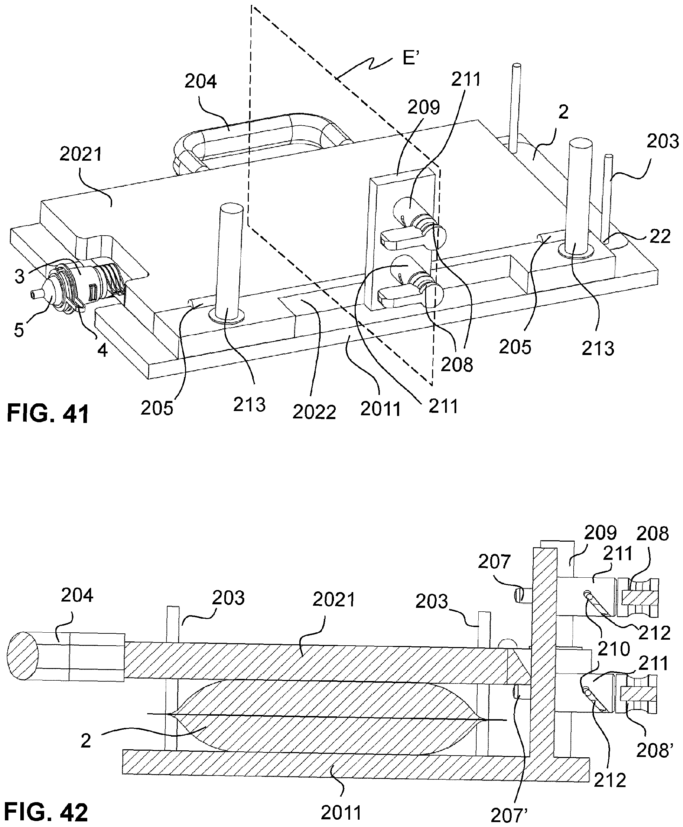

The stop mechanism and the trigger mechanism are visible in the perspective front views shown of the extraction device 20 of the first and second embodiment in Fig. 34 and Fig. 40, respectively, as well as in the perspective rear views shown in Fig. 35 and Fig. 41, and in the sectional views shown in Fig. 36 and Fig. 42. In both the first and the second embodiment, the stop mechanism comprises a first stop pin 207 and a second stop pin 207', both the first stop pin 207 and the second stop pin 207' being configured to protrude horizontally from the wall structure 209 in direction of the squeezing plate 2021 in an extended state to support the squeezing structure 202 from underneath the squeezing structure 202, and to be retractable into a retracted state in which the squeezing structure 202 is not supported and thus free to move, in particular to fall as driven by gravity. The first stop pin 207 is arranged vertically above the second stop pin 207'. In the first embodiment of the extraction device 20, the first stop pin 207 and the second stop pin 207' are arranged between the two rails 206, while in the second embodiment, the first stop pin 207 and the second stop pin 207' are arranged between the two slide posts 213.

In the start position, the squeezing structure 202 rests on the first stop pin 207, which is in the extended state, as shown in Fig. 32 and Fig. 38. In the intermediate position, the squeezing structure 202 rests on the second stop pin 207', which is in the extended state, the squeezing structure 202 being below the first stop pint 207, as shown in Figs. 33 and 36 as wells as in Figs. 39 and 42.

In order to release the squeezing structure 202 from the start position, the trigger mechanism comprises a first trigger handle 208 connected to the first stop pin 207, wherein rotating the first trigger handle 208 upwards by 90°, as indicated by the dashed arrows in Figs. 32 and 38, causes the first stop pin 207 to retract towards the wall structure 209. In order to release the squeezing structure 202 from the intermediate position, the trigger mechanism comprises a second trigger handle 208' that is connected to the second stop pin 207', wherein rotating the second trigger handle 208' upwards by 90°, as indicated by the dashed arrows in Figs. 33 and 39, causes the second stop pin 207' to retract towards the wall structure 209.

As visible in the sectional views of the extraction device 20 in Figs. 36 and 42, the stop mechanism and the trigger mechanism in the present embodiments form a bayonet-type mechanism: each of the first and second stop pin 207, 207' extends through a bayonet cylinder 211 that is fixedly arranged between the respective first and second trigger handle 208, 208' and a rear side of the wall structure 209, wherein the rear side is opposite to a front side of the wall structure 209, the front side facing the squeezing plate 2021. The bayonet cylinders 211 are attached to the rear side of the wall structure 209 so as to be non-rotatable. Each of the first and second stop pin 207,207' comprises a bayonet pin 210 extending at a right angle from the respective stop pin 207,207' and each bayonet cylinder 211 comprises a bayonet slit 212 with a first slit end and a second slit end. When the stop pin 207,207' is in the extended state, the bayonet pin 210 is located at the first slit end of the respective bayonet slit 212, the first slit end forming a notch in which the bayonet pin 210 is engaged. The first slit end is located closer to the rear side of the wall structure 209 than the second slid end. As the trigger handle 208,208' is rotated, the stop pin 207,207' rotates together with the trigger handle 208,208' and the bayonet pin moves out of the notch at the first slit end and is guided along the bayonet slit towards the second slit end, causing the stop pin 207, 207' to retract, i.e. move away from the squeezing structure 202 so as to no longer support the squeezing structure 202, which is then free to move downwards.

**LIST OF REFERENCE SIGNS**

| | | | |
|---|---|---|---|
| 1 | storage assembly | 471 | grip hole |
| 2 | flexible/inflatable bag | 5 | perforation device |
| 21 | fixation portion | 50 | perforation structure |
| 22 | fixation holes | 51 | perforation guide structure |
| 3 | input piece | 52 | connector part |
| 30 | hollow shaft portion | 53 | perforation arms |
| 31 | input alignment feature | 54 | insertion portion |
| 32 | bag connection structure | 55 | abutment ring |
| 321 | ridges | 56 | orifice |
| 322 | top part of the beak-like structure | 6 | mouthpiece |
| | | 10 | gauge device |
| 323 | bottom part of the beak-like structure | 11 | frame structure |
| | | 111 | first frame bar |
| 324 | beak tip | 112 | second frame bar |
| 33 | flap | 113 | third frame bar |
| 331 | circumferential edge region | 114 | fourth frame bar |
| 332 | disk hole | 115 | slit |
| 34 | flap attachment structure | 116 | strip |
| 340 | separation wall | 117 | fold |
| 341 | passage holes | 20 | extraction device |
| 35 | mounting feature | 201 | support structure |
| 36 | retaining notch | 2011 | support plate |
| 36' | retaining plug hole | 202 | squeezing structure |
| 37 | abutment edge | 2021 | squeezing plate |
| 4 | sealing cap | 203 | mounting posts |
| 41 | sealing membrane | 204 | handle |
| 42 | cap sleeve | 205 | hinge mechanism |
| 421 | cap guide structure | 206 | rail |
| 43 | cap alignment feature | 207 | first stop pin |
| 44 | retaining feature | 207' | second stop pin |
| 45 | retaining feature connection part | 208 | first trigger handle |
| | | 208' | second trigger handle |
| 46 | cap rim | 209 | wall structure |
| 47 | handle portion | 210 | bayonet pin |
| 211 | bayonet cylinder | 82 | mass spectrometer |
| 212 | bayonet slit | | |
| 213 | slide post | S1,S2,S3 | ruler sections |
| 214 | slide carriage | C | central axis |
| 215 | slide hole | P | pivot axis |
| 80 | analyzer assembly | | |
| 81 | ionizer device | | |

## Claims

1. A storage assembly (1) for storing a gas, preferably breath gas, comprising:
a bag (2) being inflatable and configured to contain the gas;
an input piece (3) being attached to the inflatable bag (2), the input piece (3) comprising a valve mechanism having an open valve state in which a passage for the gas through the valve mechanism is open, and a closed valve state in which the passage for the gas through the valve mechanism is closed;
a sealing cap (4) being connectable to the input piece (3), the sealing cap (4) comprising a sealing membrane (41) to seal the passage for the gas through the valve mechanism, and
a perforation device (5) being insertable into the input piece (3),
wherein the perforation device (5) comprises a perforation structure (50) configured to perforate the sealing membrane (41) when the sealing cap (4) is connected to the input piece (3) and to cause the valve mechanism to be in the open valve state once the perforation device (5) is inserted in the input piece (3).

2. The storage assembly (1) of claim 1, wherein the sealing cap (4) comprises a cap sleeve (42), the sealing membrane (41) being preferably arranged at a first end of the cap sleeve (42), wherein the input piece (3) comprises a hollow shaft portion (30) extending at least partially outside of the inflatable bag (2),
wherein the hollow shaft portion (30) is configured to at least partially receive the cap sleeve (42), and
wherein, preferably, the sealing cap (4) comprises a cap alignment feature (43) and the input piece (3) comprises an input alignment feature (31) configured to engage with the cap alignment feature (43) to enable rotational alignment of the sealing cap (4) with respect to the input piece (3).

3. The storage assembly (1) of claim 2, wherein the valve mechanism is arranged within the hollow shaft portion (30) of the input piece (3),
and wherein the sealing membrane (41) is arranged within the hollow shaft portion (30) so as to cover and thereby seal the valve mechanism when the cap sleeve (42) is at least partially received in the hollow shaft portion (30).

4. The storage assembly (1) of claim 2 or 3, wherein the perforation device (5) is at least partially insertable into the cap sleeve (42), and
wherein, preferably, the cap sleeve (42) comprises a cap guide structure (421) and the perforation device (5) comprises a perforation guide structure (51) configured to engage with cap guide structure (421) to guide the perforation device (5) when the perforation device (5) is inserted into the cap sleeve (42).

5. The storage assembly (1) of any one of the preceding claims, wherein the valve mechanism is configured as a flap valve comprising a flap (33), the flap (33) being kept in the open valve position by the perforation structure (50) once the perforation device (5) has been at least partially inserted into the input piece (3),
wherein, preferably, the perforation structure (50) comprises at least two, preferably six, perforation arms (53), the perforation arms (53) being preferably arranged equidistantly from each other on a circle.

6. The storage assembly (1) of claim 5, wherein the flap (33) is a disk defining a disk center and a circumferential edge region (331),
wherein the valve mechanism comprises a flap attachment structure (34), the disk being attached to the flap (33) attachment structure (34) at the center of the disk, and
wherein the circumferential edge region (331) is preferably in plane with the disk center in the closed valve position, and/or
wherein the circumferential edge region (331) is preferably bent towards an interior of the bag by the perforation structure (50) in the open valve position.

7. The storage assembly (1) of claim 5 and 6, wherein the perforation arms (53) and the disk are concentrically arranged, and
wherein the perforation arms (53) are bending the circumferential edge region (331) of the disk towards the interior of the bag (2) once the perforation device (5) has been at least partially inserted into the input piece (3).

8. A gauge device (10) for determining a filling state of a bag (2), preferably of the inflatable bag (2) of the storage assembly (1) according to any one of claims 1-7, the gauge device (10) comprising a frame structure (11) which defines an opening into which the bag (2) can be placed,
wherein the opening has a dimension that is adaptable to the filling state of the bag (2), and
wherein the gauge device (10) comprises a filling state marker configured to indicate the dimension of the opening.

9. The gauge device (10) of claim 8, wherein the frame structure (11) comprises a first frame bar (111) delimiting the opening on a first side and a second frame bar (112) delimiting the opening on a second side opposite to the first side,
wherein the first frame bar (111) and the second frame bar (112) are movable with respect to each other, thereby changing the dimension of the opening, and
wherein the filling state marker comprises a ruler part connected to the first frame bar (111) and a cursor part connected to the second frame bar (112), the cursor part being configured to slide along the ruler part when the first frame bar (111) and the second frame bar (112) are moving with respect to each other,
wherein, preferably, the cursor part is provided by a slit (115) in the second frame bar (112) and the ruler part is provided by a strip (116) being connected in a foldable manner to the first frame bar (111), the strip (116) being insertable into the slit (115) and slidable within the slit (115).

10. The gauge device (10) of claim 9, wherein the frame structure (11) comprises a fold (117) such that the frame structure (11) is foldable from a folded state in which the second frame bar (112) is folded onto the first frame bar (111) and the dimension of the opening is zero, to an unfolded state in which the dimension of the opening is maximized, and
wherein the gauge device (10) is preferably made of cardboard.

11. A method of delivering a gas sample, preferably a breath gas sample, to an analyzer assembly (80), the method comprising:
(1001) providing a gas sample, preferably a breath gas sample, contained in a bag (2);
(1002) placing the bag (2) in an extraction device (20), wherein the extraction device (20) comprises a support structure (201) and a squeezing structure (202) being arranged such that the bag (2) is received between the support structure (201) and the squeezing structure (202);
(1003) establishing a gas-tight connection between the bag (2) and the analyzer assembly (80), the analyzer assembly (80) preferably comprising an ionizer device (81) connected to a mass spectrometer (82);
(1007) applying pressure onto the bag (2) via the squeezing structure (202) to cause the gas sample to flow from the bag (2) into the analyzer assembly (80).

12. The method of claim 11,
wherein the gas sample is provided in the storage assembly (1) according to any one of claims 1-7, the bag (2) being the inflatable bag (2) of said storage assembly (1), wherein the sealing cap (4) is connected to the input piece (3) so as to seal the valve mechanism,
wherein the method further comprises:
(1004) inserting the perforation device (5) into the sealing cap (4);
(1005) perforating the sealing membrane (41) with the perforation structure (50);
(1006) forcing the valve mechanism into the open valve state with the perforation structure (50) to enable the gas sample to flow from the inflatable bag (2) into the analyzer assembly (80);
wherein, preferably, the perforation device (5) is insertable into the sealing cap (4) such that a gas-tight connection between the perforation device (5) and the sealing cap (4) is formed prior to perforating the sealing membrane (41), and
wherein, preferably, the perforation device (5) comprises a connector part (52) and the step (1003) of establishing a gas-tight connection between the inflatable bag (2) and the analyzer assembly (80) comprises:
establishing a gas-tight connection between the connector part (52) of the perforation device (5) and the analyzer assembly (80), preferably using a tube (83).

13. The method of claim 11 or 12, wherein the method further comprises:
(1008) interrupting the application of pressure onto the bag (2) after a first predetermined portion of the gas sample has been extracted from the bag (2);
(1009) switching a setting of the analyzer assembly (80), in particular in case the analyzer assembly (80) comprises an ionizer device (81), switching the ionizer device (81) between a positive ionization mode and a negative ionization mode, and
(1010) resuming the application of pressure onto the bag (2) to cause a second predetermined portion of the gas sample to flow into the analyzer assembly (80).

14. An extraction device (20) for controlled extraction of a gas from a bag (2), preferably for use in the method of claims 11-13, the extraction device (20) comprising:
a support structure (201), and
a squeezing structure (202),
wherein the support structure (201) and the squeezing structure (202) are arranged such that the bag (2), preferably being the inflatable bag (2) of the storage assembly (1) of claims 1-7, can be received between the support structure (201) and the squeezing structure (202),
wherein the squeezing structure (202) is movable with respect to the support structure (201) from a start position to an end position, and
wherein the squeezing structure (202) is configured to exert pressure onto the bag (2) while moving, preferably in a translatory movement, from the start position to the end position when the bag (2) is received between the support structure (201) and the squeezing structure (202) to cause the gas contained in the bag (2) to exit the bag (2).

15. The extraction device (20) of claim 14, further comprising:
a stop mechanism configured to stop the squeezing structure (202) from exerting pressure onto the bag (2) after the squeezing structure (202) has moved from the start position to an intermediate position located between the start position and the end position, and
a trigger mechanism configured to unlock the stop mechanism and thereby release the squeezing structure (202) from the intermediate position.
